(19) Europäisches Patentamt
European Patent Office
Office européen des brevets

(11) Publication number: **0 148 612 A2**

(12) EUROPEAN PATENT APPLICATION

(21) Application number: **84308765.1**

(22) Date of filing: **14.12.84**

(51) Int. Cl.⁴: **C 12 P 17/12**
// (C12P17/12, C12R1:72)

(30) Priority: **10.01.84 US 569689**

(43) Date of publication of application: **17.07.85**
**Bulletin 85/29**

(84) Designated Contracting States: **AT BE CH DE FR GB IT LI LU NL SE**

(71) Applicant: **AYERST, MCKENNA AND HARRISON INC.,**
**1025 Laurentian Boulevard, St. Laurent Quebec H4R 1J6 (CA)**

(72) Inventor: **Sehgal, Surendra Nath, 4 Sayre Drive, Princeton New Jersey 08540 (US)**
Inventor: **Bagli, Jehan Framroz, 7 Cleveland Lane, RD 4, Princeton New Jersey 08540 (US)**

(74) Representative: **Brown, Keith John Symons et al, c/o John Wyeth & Brother Limited Patent and Trademark Department Huntercombe Lane South, Taplow Maidenhead Berkshire SL6 0PH. (GB)**

(54) **A stereospecific microbial reduction.**

(57) The dopaminergic antagonist ciladopa, also known as S(-)-2-[4-[2-hydroxy-2-(3,4-dimethoxyphenyl)ethyl]-1-piperazinyl]-2,4,6-cycloheptatrien-1-one, is prepared by stereospecific microbial reduction of 2-[4-[2-(3,4-dimethoxyphenyl)-2-oxoethyl]-1-piperazinyl]-2,4,6-cycloheptatrien-1-one.

EP 0 148 612 A2

This invention relates to ciladopa and to a microbiological process for its production.

Ciladopa is the generic name of the pharmacologically active enantiomer S(-)-2-[4-[2-hydroxy-2-(3,4-dimethoxyphenyl)ethyl]-1-piperazinyl]-2,4,6-cycloheptatrien-1-one. The compound is a dopaminergic antagonist and is indicated for the treatment of, inter alia, Parkinson's disease and hyperprolactinemia. The preparation by chemical means and the properties of ciladopa are described by J.F.Bagli, T.Bogri and K.Voith in Canadian Patent 1,153,764, issued September 13, 1983, and in the corresponding published European Patent Application 0034894-A2. A preferred mode of preparation, as described in the documents, is the reduction by sodium borohydride of "the precursor ketone", namely 2-[4-[2-(3,4-dimethoxyphenyl)-2-oxoethyl]-1-piperazinyl]-2,4,6-cycloheptatrien-1-one, followed by resolution of the racemic reduction product to give ciladopa.

It is an object of this invention to provide ciladopa by stereoselective reduction of the precursor ketone, thereby advantageously eliminating the need to perform a cumbersome resolution step. According to the present invention, this attractive alternative is realized by exposing the precursor ketone to the reductive enzymatic action of a microorganism. The microorganism is characterized by its ability to produce an enzyme capable of reducing the aliphatic keto group of the precursor (or substrate) ketone to a hydroxy group having the required configuration. Moreover, this new reduction process is enantioselective i.e. it produces the optically pure enantiomer, avoids obnoxious reagents and is amenable to commercial application. The stereoselectivity of the reduction process of the present invention is surprising in view of the K. Horikoshi et al. reference, cited below.

Stereoselective microbial reductions of organic ketones have been reported; for example, Ciba A.G.

Swiss Patent 366,530, February 28, 1963; Farmdoc 44700S for Noda Sangyo Kaguku Kenkyujo Japanese Patent 7123038-R, July 1, 1971; F.W.Kunstmann et al., East German Patentschrift 64054, published October 5, 1968; Syntex Corporation Netherland Patent 7012270, February 24, 1971; J.R.McMullen, U.S.Patent 4,004,978, January 25,1977; and K. Kieslich et al., U.S.Patent 4,247,635, January 27, 1981. However, each new situation presents a different set of problems to overcome. The previously reported reductions are readily distinguished from the process of the present invention in that different substrates and different microorganisms are used.

A publication also exists in which a strain of Candida guilliermondii has been used to reduce the keto group of 2-methyl-3-(2-furyl)-3-oxopropionic acid, methyl ester, to a hydroxy group. See K. Horikoshi et al., Agric. Biol. Chem., 47,435 (1983); Chem. Abstr., 98, 175952n (1983). The reduction, however, was not stereoselective.

The present invention relates to a process for preparing optically pure ciladopa by the microbiological reduction of the substrate ketone, 2-[4-[2-(3,4-dimethoxyphenyl)-2-oxoethyl]-1-piperazinyl]-2,4,6-cycloheptatrien-1-one, or an acid addition salt thereof. This invention particularly provides a process for preparing optically pure ciladopa, which comprises cultivating an enzyme-producing microorganism in a nutrient fermentation medium to produce the enzyme, the enzyme being capable of reducing the aliphatic keto group of 2-[4-[2-(3,4-dimethoxyphenyl)-2-oxoethyl]-1-piperazinyl]-2,4,6-cycloheptatrien-1-one to a hydroxy group of the required configuration, and exposing 2-[4-[2-(3,4-dimethoxyphenyl)-2-oxoethyl]-1-piperazinyl]-2,4,6-cycloheptatrien-1-one, or an acid addition salt thereof, to the enzyme to reduce 2-[4-[2-(3,4-dimethoxyphenyl)-2-oxoethyl]-1-piperazinyl]-2,4,6-cycloheptatrien-1-one, or an acid addition salt thereof, to ciladopa.

A preferred microorganism for preparing optically active ciladopa by microbial reduction of the substrate ketone is a strain of the genus *Candida*, e.g. *Candida guilliermondii*.

In an embodiment of this invention, the production of optically pure ciladopa is realized by cultivating the microorganism *Candida guilliermondii* in a nutrient medium under aerobic conditions until a substantial concentration of microorganism cells is achieved, and thereafter transforming the substrate ketone, or an acid addition salt thereof, to ciladopa by microbially reducing the precursor ketone, or the acid addition salt thereof, with the cells so obtained.

A preferred example for preparing optically pure ciladopa comprises cultivating the microorganism *Candida guilliermondii* in an aqueous nutrient medium under submerged aerobic conditions until a substantial concentration of microorganism cells is achieved; adding 2-[4-[2-(3,4-dimethoxyphenyl)-2-oxoethyl]-1-piperazinyl]-2,4,6-cycloheptatrien-1-one, or an acid addition salt thereof, to the aqueous nutrient medium; incubating the resultant mixture until substantial amounts of ciladopa is present in the mixture; and isolating ciladopa from the mixture.

The stereoselective process is represented schematically as follows:

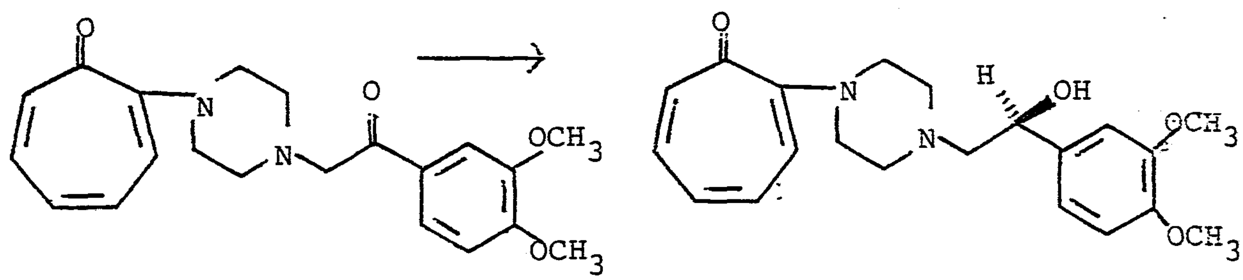

The "precursor ketone" or "substrate ketone", can be prepared according to procedures described in the aforementioned Canadian Patent 1,153,764 and the aforementioned European Application 0034894-A2. The acid addition salts thereof are prepared by conventional methods. A preferred acid addition salt is the hydrochloric acid addition salt, IR (white mineral oil)1670,1585,1555,1010,969 $cm^{-1}$.

The term "aliphatic keto group", with reference to the substrate ketone, means the keto group attached to the carbon atom adjacent to the phenyl group present in the substrate ketone, namely 2-[4-[2-(3,4-dimethoxyphenyl)-2-oxoethyl]-1-piperazinyl]-2,4,6-cycloheptatrien-1-one.

A suitable strain of microorganism for the present process is preferably a strain of the genus Candida which is capable of producing the aforementioned keto-reducing enzyme. Readily available strains for this purpose, available without restrictions, are Candida guilliermondii ATCC 9058 and Candida guilliermondii ATCC 9390, microorganisms which are part of the permanent collection of the American Type Culture Collection (ATCC), 1230 Parklawn Drive, Rockville, Maryland, 20852, U.S.A.

Notwithstanding the suitability of Candida guilliermondii ATCC 9058 or Candida guilliermondii ATCC 9390 for the present process, it is to be understood that the invention is not limited to the use of those particular strains of microorganisms, but includes the use of other suitable microorganisms, variations and mutants thereof, obtained by natural selection or by treatment of the microorganism with, for instance, ultraviolet rays, X-rays, N-methyl-N-nitro-N-nitrosoguanidine, manganese chloride, camphor, nitrogen mustards, and the like, as well as polyploids of the various mutants.

Candida guilliermondii is advantageously grown and stored on a suitable aga slant culture. The composition of the medium used for the slant culture is not critical so long as it is sterile and contains adequate nutrients and growth factors to support the microorganism.

With reference to the process, two phases are involved, mainly cell production and transformation (bioconversion).

The cell production can be accomplished under conditions similar to those used generally for aerobic fermentation processes. Thus, for example, cell production can be achieved under submerged aerobic conditions in an aqueous nutrient medium inoculated with the microorganism as provided, for example, by a fresh slant of Candida guilliermondii. The inoculated medium can be incubated in flasks placed on shaking machines. For industrial production, metal tanks can be substituted. Cell growth can also be achieved by surface cultivation. The microorganism requires as nutrient elements assimilable carbon and organic nitrogenous substances. The presence of mineral salts is desirable. Cultivation is best effected when the initial pH of the culture medium is between 4 and 8, the optimum pH being around 4.5 - 5.5.

The utilizable sources of assimilable carbon for the production of the microbial cells are very diverse, there being included sugars, such as D-glucose, D-xylose and D-fructose, dextrin, starches of different types of origin, glycerol and (other polyalcohols), inositol and animal and vegetable fats, as well as esters thereof. The sources of organic assimilable nitrogen are substances, such as malt extract, various peptones, soybean meal, cotton meal and other vegetable meals (whole, partially or totally defatted), meat flours or animal viscera, casein hydrolystates, soybean hydrolysates, yeast hydrolysates, lactalbumin, wheat glutins, distillers solubles, corn steeps, molasses, urea and amino acids.

Mineral salts, such as the chlorides, nitrates, sulfates, carbonates and phosphates of sodium, potassium, ammonium and calcium, may be included in appropriate concentrates. The nutritive medium also may contain a number of trace elements such as magnesium, iron, manganese, and zinc.

Under the above conditions suitable enzyme-producing cell concentration is obtained in 24 to 48 hours at temperatures ranging from 25 to 35°C, preferably 27 to 30°C.

The actual enantioselective reduction, or transformation, of the substrate ketone is accomplished by exposing it or bringing it into contact with the reductive enzyme action of the cells in accordance with conventional microbiological methodology. In one embodiment, the substrate ketone, or acid addition salt thereof, is dissolved in a suitable solvent, for example, methanol, ethanol, dimethylformamide or dimethylsulfoxide. The resultant solution is added to the above mentioned nutrient medium containing a suitable concentration of growing cells. The resultant mixture is incubated under aerobic conditions until substantial amounts of ciladopa is present in the mixture.

Alternatively, the transformation is performed with resting cells or with immobilized cells. The resting cells can be obtained by harvesting the cells from the cell production nutrient medium by centrifugation. The immobilized cells can be obtained by subsequent entrapment of the resting cells in a polymeric matrix, or by entrapment in alginate beads of the growing cells in the nutrient medium. Thereafter, the resting cells or the immobilized cells are suspended in a suitable transformation medium, preferably an aqueous buffered medium (pH = 4 to 8); for example, a sodium phosphate buffer containing glucose and a suitable defoaming agent. A solution or suspension of the substrate ketone in water is added to the cell suspension in the buffer medium and the resultant mixture is incubated until substantial amounts of ciladopa is present.

Still another mode of transformation involves the use of the isolated enzyme, instead of the cells, in the transformation medium. The enzyme is isolated by rupturing the resting cells and separating the freed enzyme from the ruptured cells according to microbiological methodology.

The transformation, in any case, is best achieved under aerobic conditions within 24 to 72 hours when the pH of the transformation medium is kept between 4 and 8, the optimum pH being around 4.5 to 5.5, and a temperature ranging from 25 to 45°C, preferably from 35 to 40°C. The best yields of the transformation product are obtained with a charge of about three to six, preferably four, grams of the substrate ketone per litre of the transformation medium. Antibiotics, such as neomycin or chloramphenicol, optionally may be used to retard bacterial contamination.

Thereafter, a variety of procedures may be employed in the isolation and purification of the product; for example, solvent extraction, partition chromatography, silica gel chromatography, and crystallization from solvents. Solvent extraction procedures, followed by crystallization, are preferred for commercial recovery inasmuch as they are less time consuming and less expensive.

Immobilized cells can be reharvested and re-used for subsequent transformations for at least four more times.

The following example illustrates further this invention:

EXAMPLE

(a) Microorganism: *Candida guilliermondii* ATCC 9390 was grown and maintained on Sabouraud-dextrose agar medium. Good growth was obtained after 48 hours of incubation at 28°C

First Stage Inoculum: An aqueous inoculum medium (500 mL) consisting of (g/L): soy peptone, 10; glucose (cerelose), 20; malt extract, 50; $KH_2PO_4$, 2; was sterlized at 121°C for 30 minutes in a 2L Erlenmeyer flask. The cooled medium was inoculated with a suspension of cells obtained from an agar slant of *Candida guilliermondii* ATCC 9390 and incubated at 30°C for 24 hours on a rotary shaker at 250 rev/min (2", i.e. 5.08 cm, stroke).

Cell production: An aqueous nutrient fermentation medium (20 L) consisting of (g/L): soy peptone, 10; glucose, 50; malt extract, 10; and 0.25 mL of antifoam Mazur DF-287 (Mazur Chemical Corp; Gurnee, Illinois, U.S.A.); was sterilized in a 28 L fermenter at 121°C for 30 minutes. After cooling, the medium was inoculated with 500 mL of inoculum. The incubation was done at 30°C with agitation

of 300 rev/min and aeration of 0.25 V/V/min.

After 24 hours of incubation, the cells were flocculated by adding 150 g of sodium alginate (0.75% W/V in water) to the broth in the fermenter and raising the temperature to 40°C. The alginate broth mixture was agitated for at least 15 minutes. This mixture was then forced by air pressure through an orifice (¼",i.e. 0.64 cm, diameter) into 2 L of 1.1 M $CaCl_2$ solution cooled to 0°C and kept in an ice bath. During the addition of the alginate/broth mixture, the calcium chloride solution was stirred constantly.

The flocculated/immobilized cells were recovered in a Buchner funnel over cheese cloth and washed thoroughly with sterile distilled water. A 20 L broth run yielded about 4 kg (wet weight) of alginate-immobilized cells.

Transformation: The immobilized cells (4 kg) were resuspended in 19 L of an aqueous medium consisting of (g/L): $CaCl_2$, 11.1; glucose, 20; and the antifoam DF-287, 0.25 mL. In a 28 L fermenter, 80 g of 2-[4-[2-(3,4-dimethoxyphenyl)-2-oxoethyl]-1-piperazinyl]-2,4,6-cycloheptatrien-1-one hydrochloride, suspended in a sufficient amount of distilled water (ca. 1L) for transfer purposes, was added to the cell suspension. The transformation was done at 37°C with agitation of 300 rev/min and aeration of 0.25 V/V/min. The pH was controlled at 5.0 with on-demand addition of $NH_3$ gas. Complete transformation of ketone to alcohol took place in 65 hours, as determined by thin layer chromatography examination of the extract.

The immobilized cells were filtered over cheese cloth and washed thoroughly with water. The filtrate and the washings were extracted for product recovery as described below. The immobilized cells were resuspended in transformation medium and another transformation run was initiated. The immobilized cells were used 4 times. Complete transformation of starting material was obtained

in 48 hours in the re-use runs.

Product recovery: The pH of the filtrate and washings was adjusted to 8.5. The two solutions were combined and extracted twice with ½ volume of methylene chloride. The agitation time for each extraction was 30 minutes. The extracts were combined (total volume 30 L), reduced under vacuum to a smaller volume (10 L), dried with anhydrous sodium sulfate and concentrated to about 500 mL. About 2L of hexane was added slowly to the stirred concentrate. The resultant precipitate was collected to give 60 g of optically pure ciladopa. Crystallization of a portion of the latter product from diethyl ether gave a pure sample of ciladopa with mp 122-124°C and $[\alpha]_D^{25}$-12.0°C (c = 1, dimethylformamide). Identity was confirmed by microanalysis and by IR, UV and NMR spectroscopy.

(b) The substitution of Candida guilliermondii ATCC 9058 for Candida guilliermondii ATCC 9390 in the preceding process also resulted in the stereoselective reduction of 2-[4-[2-(3,4-dimethoxyphenyl)-2-oxoethyl]-1-piperazinyl]-2,4,6-cycloheptatrien-1-one hydrochloride to ciladopa.

CLAIMS

1. A process for preparing optically pure ciladopa, which comprises cultivating an enzyme-producing microorganism in a nutrient fermentation medium to produce the enzyme, the enzyme being capable of reducing the aliphatic keto group of 2-[4-[2-(3,4-dimethoxyphenyl)-2-oxoethyl]-1-piperazinyl]-2,4,6-cycloheptatrien-1-one to a hydroxy group of the required configuration, and exposing 2-[4-[2-(3,4-dimethoxyphenyl)-2-oxoethyl]-1-piperazinyl]-2,4,6-cycloheptatrien-1-one, or an acid addition salt thereof, to the enzyme to reduce 2-[4-[2-(3,4-dimethoxyphenyl)-2-oxoethyl]-1-piperazinyl]-2,4,6-cycloheptatrien-1-one, or an acid addition salt thereof, to ciladopa.

2. A process as claimed in Claim 1 wherein the enzyme-producing microorganism is a strain of the genus *Candida*.

3. A process as claimed in Claim 1 wherein the microorganism is *Candida guilliermondii*.

4. A process for preparing optically pure ciladopa, which comprises cultivating the microorganism <u>Candida guilliermondii</u> in a nutrient medium under aerobic conditions until a substantial concentration of microorganism cells is achieved, and thereafter transforming the substrate ketone, 2-[4-[2-(3,4-dimethoxyphenyl)-2-oxoethyl]-1-piperazinyl]-2,4,6-cycloheptatrien-1-one, or an acid addition salt thereof, to ciladopa by microbially reducing the substrate ketone, or an acid addition salt thereof, with the cells so obtained.

5. A process as claimed in Claim 4 wherein the cultivation is performed at a temperature from 25 to 35°C at an initial pH from 4 to 8.

6. A process as claimed in Claim 4 or 5 wherein the transformation is performed at a temperature from 25 to 45°C at a pH from 4 to 8.

7. A process as claimed in Claim 6 wherein the transformation is performed in a transformation medium having a charge of the substrate ketone ranging from 3 to 5 g of the substrate ketone per litre of the medium.

8. A process as claimed in Claim 4 wherein the transformation is effected with resting or immobilized cells of <u>Candida guilliermondii</u>.

9. A process as claimed in any one of Claims 1 to 8 in which 2-[4-[2-(3,4-dimethoxyphenyl)-2-oxoethyl]-1-piperazinyl]-2,4,6-cycloheptatrien-1-one is employed in the form of its acid addition salt with hydrochloric acid.

10. A process for preparing optically pure ciladopa, which comprises cultivating the microorganism Candida guilliermondii in an aqueous nutrient medium under submerged aerobic conditions, adding 2-[4-[2-(3,4-dimethoxyphenyl)-2-oxoethyl]-1-piperazinyl]-2,4,6-cycloheptatrien-1-one, or an acid addition salt thereof, to the aqueous nutrient medium; incubating the resultant mixture; and isolating ciladopa from the incubated mixture.

11. A process as claimed in any one of Claims 1 to 10 in which the microorganism is Candida guilliermondii ATCC 9058 or Candida guilliermondii ATCC 9390.